# EUROPEAN PATENT APPLICATION

(11) **EP 1 683 784 A1**
(43) Date of publication of application: **26.07.2006**
(21) Application number: 04773029.6
(22) Date of filing: 14.09.2004
(51) Int. Cl.: C07C 251/30, C09B 55/00, B41M 5/30, G02B 5/22, G11B 7/24

(54) **DIIMONIUM SALT COMPOUND AND USE THEREOF**

(30) Priority: 10.11.2003 JP 2003379983; 28.04.2004 JP 2004133018
(71) Applicant: NIPPON KAYAKU KABUSHIKI KAISHA, Tokyo 102-8172 (JP)
(72) Inventor: IKEDA, Masaaki, NIPPON KAYAKU KABUSHIKI KAISHA, Tokyo 1150042 (JP); KURATA, Takaaki, NIPPON KAYAKU KABUSHIKI KAISHA, Tokyo 1150042 (JP); TORINIWA, Toshitaka, NIPPON KAYAKU KABUSHIKI KAISH, Tokyo 1150042 (JP)
(74) Representative: Wablat, Wolfgang
(86) International application number: PCT/JP2004/013347
(87) International publication number: WO 2005/044782

(57) **Abstract**

To provide a near-IR absorption compound free from antimony or arsenic and excellent in stability, especially, in heat resistance, light fastness, and moisture-and-heat resistance and also an IR absorption filter, an optical information recording medium, and a resin composition excellent in durability by using the near-IR absorption compound.

The near-IR absorption compound is a diimmonium compound having the following structure and the resin composition contains the diimmonium compound: 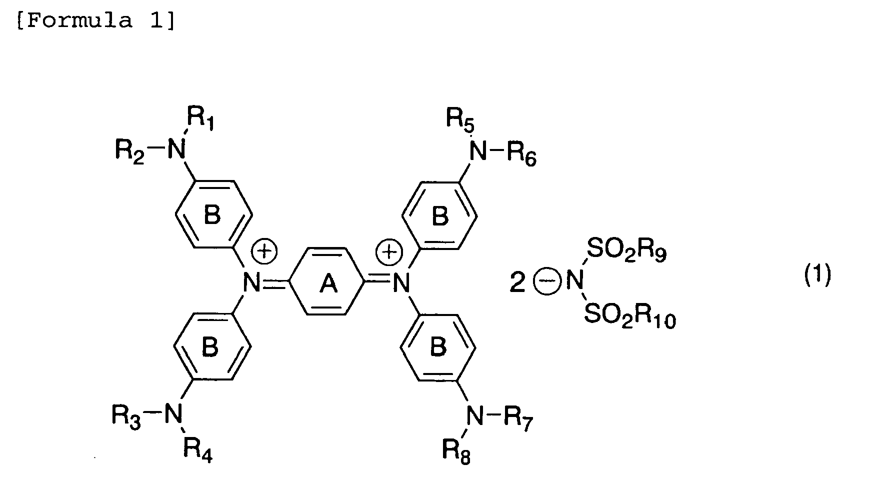 (wherein R₁ to R₈ independently denote hydrogen atom or an optionally substituted aliphatic hydrocarbon group; R₉ and R₁₀ independently denote an aliphatic hydrocarbon group optionally containing a halogen atom; and rings A and B may further have substituent groups.).

## Description

### Technical Field

The invention relates to a diimmonium compound having absorption in the infrared (IR) region and its use. Particularly, the invention relates to a diimmonium compound which is not a toxic substance and excellent in heat resistance, light resistance, and solubility and has a widened range of application, an IR absorption filter, an optical information recording medium, and a resin composition of the compound.

### Background Art

Conventionally, diimmonium compounds as near-infrared (near-IR) ray absorbers have been known widely (e.g. refer to Patent Document Nos. 1 to 3) and employed for near-IR absorption filters, heat insulation films, and sunglasses. However, among these compounds, those comprising hexafluoroantimonate ion and hexafluoroasrenic ion as counter ions are excellent in heat resistance and above all, those comprising hexafluoroantimonate ion as a counter ion have mainly been used. However, since compounds comprising antimony are appointed as toxic substances due to the inclusion of antimony, it has been desired in recent years to develop those which are free from such metals in industrial fields, especially in electric material fields, where use of heavy metals is restricted. As means of solving the above-mentioned problems, there are methods of using perchlorate ion, hexafluorophosphate ion, borofluoride ion, and the like, however, in terms of heat resistance and moisture-and-heat resistance, these counter ions are insufficient. Also, compounds comprising organic counter ions such as naphthalenedisulfonic acid have been proposed (e. g. refer to Patent Document No.2), such compounds have low molar absorption coefficients and are slightly green and therefore they cannot be employed for practical use. Further, compounds comprising trifluoromethanesulfonate ion have been known (e.g. refer to Patent Document No. 1).
Patent Document No. 1: Japanese published examined patent application No. Hei 7-51555 (P.2)
Patent Document No. 2: Japanese published unexamined patent application No. Hei 10-316633 (P.5)
Patent Document No. 3 : Japanese published examined patent application No. Sho 43-25335 (P.7 to P.14)

### Disclosure of Invention

In such a situation as described above, the invention has been accomplished and the aim of the invention is to provide a near-IR absorption compound free from antimony and excellent in stability, especially, in heat resistance, light resistance, and moisture-and-heat resistance and also solubility and therefore having a widened range of application fields; an IR absorption filter (particularly for plasma display panels) produced from the near-IR absorption compound; and an optical information recording medium and a resin composition excellent in durability.

Based on dedicated efforts made to solve the above-mentioned problems, the inventors of the invention have found that a near-IR absorption compound having a structure defined by the following structural formula (1) meets the above-mentioned aim and have accomplished the invention.

That is, the invention relates to:
(1) a diimmonium compound having a structure defined by the following general formula (1); 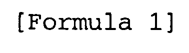 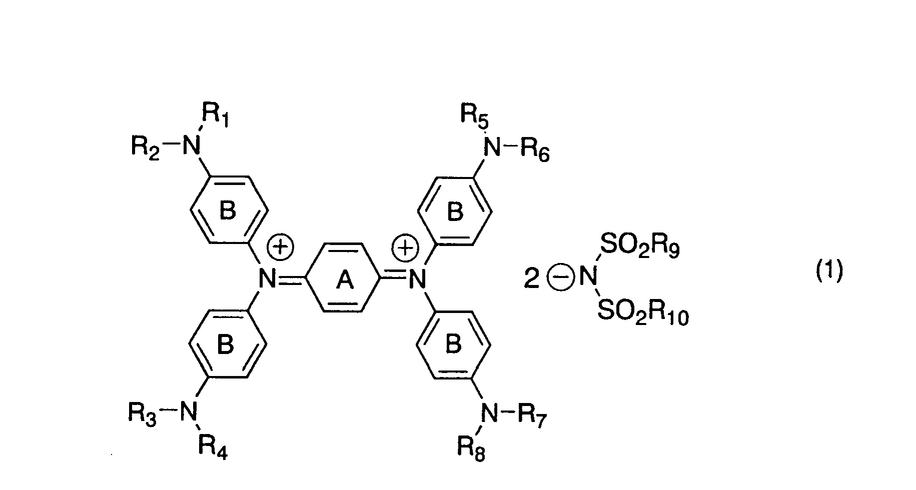 wherein R₁ to R₈ independently denote hydrogen atom or an optionally substituted aliphatic hydrocarbon group; R₉ and R₁₀ independently denote an aliphatic hydrocarbon group optionally containing a halogen atom; and rings A and B may further have substituent groups;
(2) a diimmonium compound as described in (1), of which the substituent groups of the optionally substituted aliphatic hydrocarbon groups denoted as R₁ to R₈ of the general formula

(1) are independently a halogen atom, a cyano group, a nitro group, a hydroxyl group, a carboxyl group, a carboxyamido group, an alkoxycarbonyl group, an acyl group, an aryl group, or an alkoxyl group;
(3) a diimmonium compound as described in (1) or (2), of which R₉ and R₁₀ of the general formula (1) are aliphatic hydrocarbon groups containing a fluorine atom;
(4) a diimmonium compound as described in one of (1) to (3), of which R₉ and R₁₀ of the general formula (1) are trifluoromethyl;
(5) a diimmonium compound as described in one of (1) to (4), of which at least one of R₁ to R₈ of the general formula (1) is an aliphatic hydrocarbon group comprising a cyano group;
(6) a diimmonium compound as described in one of (1) to (4), of which at least one of R₁ to R₈ of the general formula (1) is an aliphatic hydrocarbon group comprising a cyano group and at least one of them is an aliphatic hydrocarbon group comprising no cyano group;
(7) a diimmonium compound as described in one of (1) to (4), of which at least one of R₁ to R₈ of the general formula (1) is an aliphatic hydrocarbon group comprising a halogen atom;
(8) a diimmonium compound as described in one of (1) to (4), of which at least one of R₁ to R₈ of the general formula (1) is an aliphatic hydrocarbon group comprising an alkoxy group;
(9) a diimmonium compound as described in one of (1) to (4), of which at least one of R₁ to R₈ of the general formula (1) is an aliphatic hydrocarbon group comprising an aryl group;
(10) a diimmonium compound as described in one of (1) to (4), of which at least one of R₁ to R₈ of the general formula (1) is a C1 to C6 straight chain alkyl group;
(11) a diimmonium compound as described in one of (1) to (4), of which at least one of R₁ to R₈ of the general formula (1) is a C1 to C3 straight chain alkyl group;
(12) a diimmonium compound as described in one of (1) to (4), of which at least one of R₁ to R₈ of the general formula (1) is a branched alkyl group;
(13) a diimmonium compound as described in one of (1) to (4), of which all of R₁ to R₈ of the general formula (1) are alkyl groups branched at terminals;
(14) a diimmonium compound as described in one of (1) to (4), of which R₁ to R₈ of the general formula (1) are iso-butyl or iso-amyl;
(15) a diimmonium compound as described in one of (1) to (4), of which at least one of R₁ to R₈ of the general formula (1) is an unsaturated aliphatic hydrocarbon group;
(16) a composition containing the diimmonium compound described in one of (1) to (15);
(17) a near-IR absorption filter comprising a layer containning the diimmonium compound described in one of (1) to (15) ;
(18) a near-IR absorption filter for plasma displays comprising a layer containing the diimmonium compound described in one of (1) to (15);
(19) an optical information recording medium comprising a recording layer containing the diimmonium compound described in one of (1) to (15);
(20) an optical information recording medium comprising a recording layer containing the diimmonium compound described in one of (1) to (15) and an organic dye selected from a group consisting of cyanine type dyes, squarylium type dyes, indoaniline type dyes, and polymethine type dyes;
(21) a resin composition containing the diimmonium compound described in one of (1) to (15);
(22) a near-IR absorption compound being a salt comprising a cation obtained by oxidizing a compound having the following general formula (2) and an anion having the following general formula (3) and necessary for neutralizing the cation; 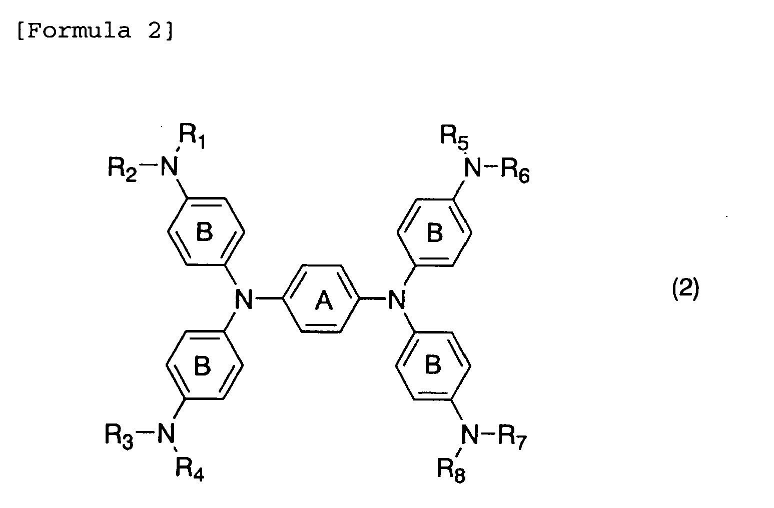 wherein R₁ to R₈ independently denote hydrogen atom or an optionally substituted aliphatic hydrocarbon group; and rings A and B may further have substituent groups; 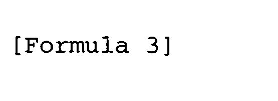 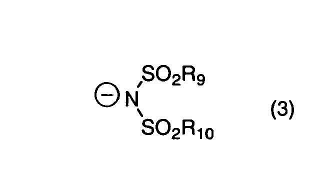 wherein R₉ and R₁₀ independently denote an aliphatic hydrocarbon group optionally containing a halogen atom;
(23) a diimmonium compound having the following general formula (5) 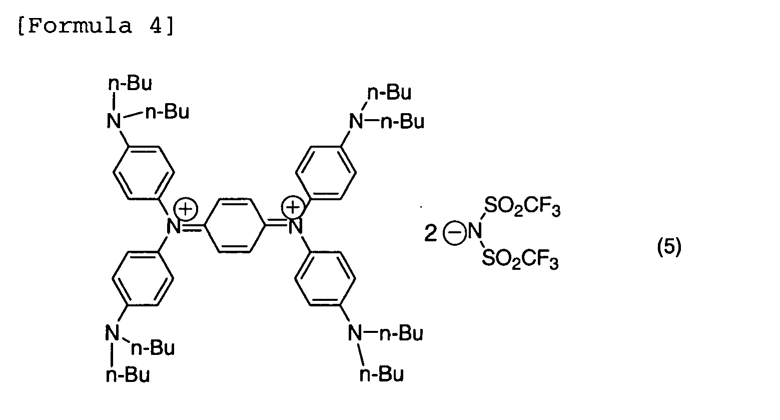 wherein n-Bu stands for n-butyl;
(24) a diimmonium compound as described in (23) having the maximum absorption wavelength (λmax) (measured in dichloromethane) of 1,102 nm;
(25) a diimmonium compound having the following general formula (6) 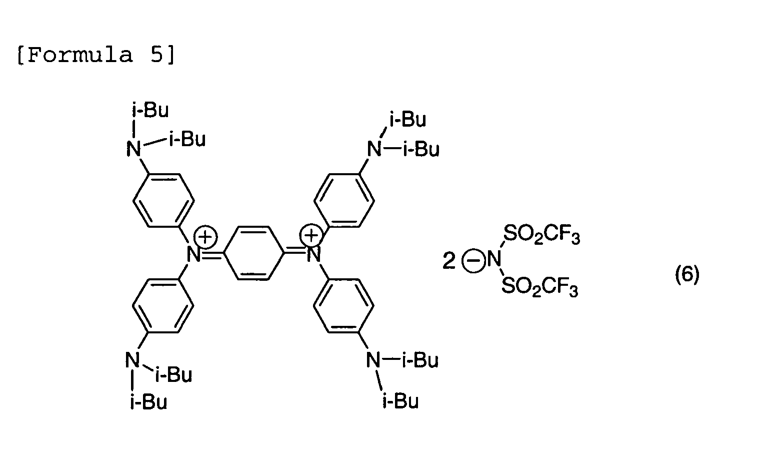 wherein i-Bu stands for iso-butyl;
(26) a process for the preparation of a diimmonium compound having the following general formula (1) 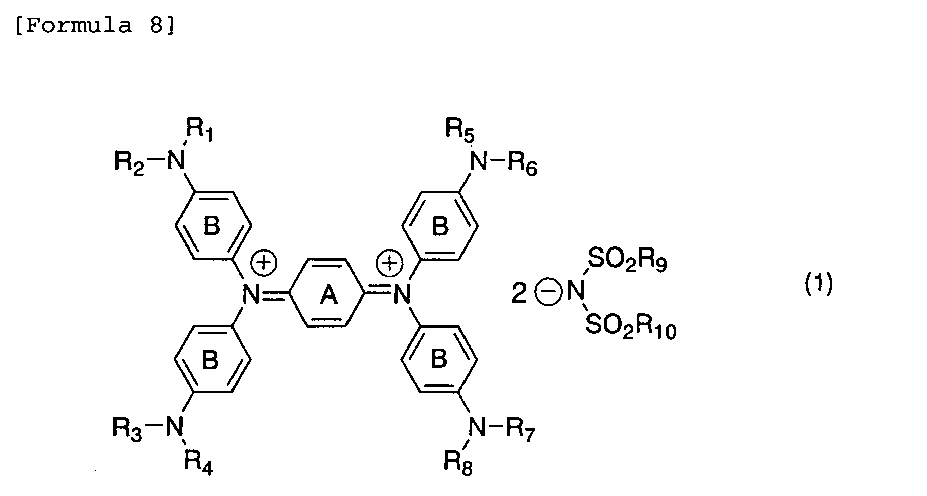 wherein R₁ to R₈ independently denote hydrogen atom or an optionally substituted aliphatic hydrocarbon group; R₉ and R₁₀ independently denote an aliphatic hydrocarbon group optionally containing a halogen atom; and rings A and B may further have substituent groups by carrying out oxidation reaction by adding a silver salt of a mineral acid and an alkali metal salt of an anion having the following general formula (3) 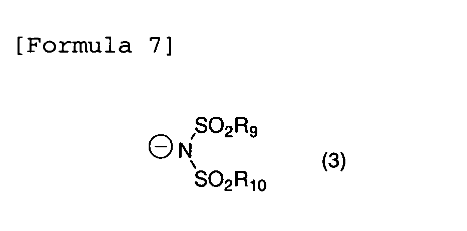 wherein R₉ and R₁₀ are the same as defined above to a phenylenediamine compound having the following general formula (2) 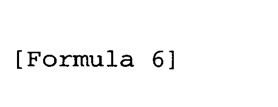 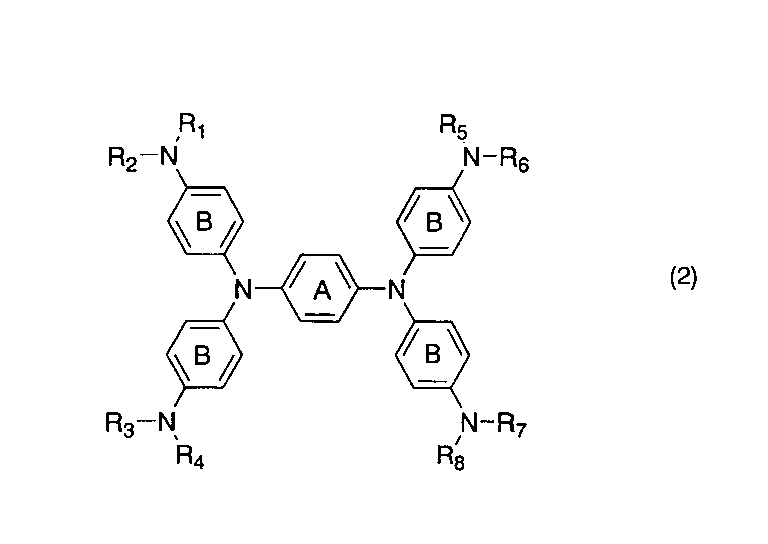 wherein R₁ to R₈ and rings A and B are the same as defined above;
(27) a process for the preparation of a diimmonium compound as described in (26) of which all of R₁ to R₈ are the same group selected from a group consisting of ethyl, n-butyl, iso-butyl, iso-amyl, 3-cyano-n-propyl, and 4-cyano-n-butyl and R₉ and R₁₀ are both trifluoromethyl;
(28) a process for the preparation of a diimmonium compound as described in (26) or (27) in which the silver salt of a mineral acid to be used is silver nitrate and the alkali metal salt of an anion to be used is a potassium salt;
(29) a process for the preparation of a diimmonium compound as described in one of (26) to (28) in which the reaction is carried out in a water-soluble polar solvent.

### Best Modes for Carrying Out the Invention

The diimmonium compound of the invention comprises a diimmonium cation and two di (alkylsulfonyl) imido anion salts as counter ions and is defined by the following general formula (1):

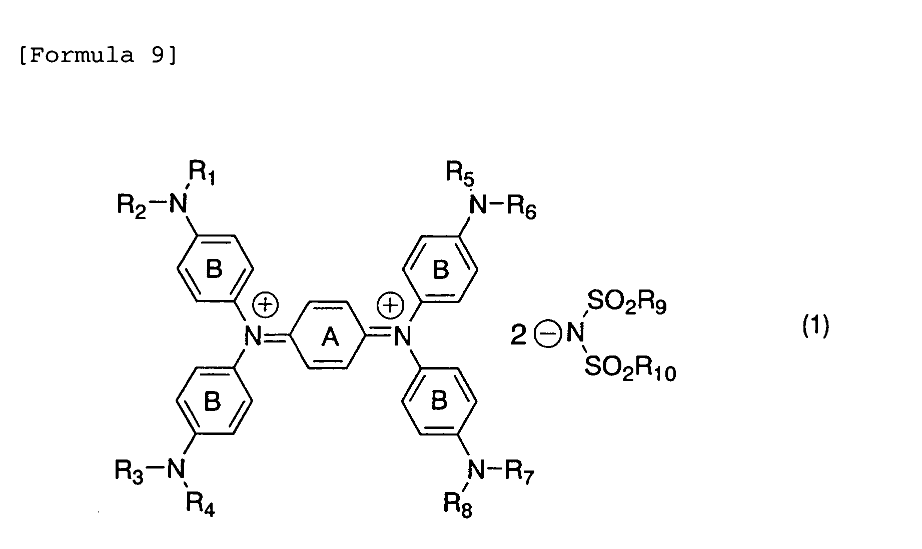

In the general formula (1), R₉ and R₁₀ independently denote an aliphatic hydrocarbon group optionally containing a halogen atom. As the aliphatic hydrocarbon group, preferable examples are saturated and unsaturated, straight chain, branched, and cyclic alkyl groups comprising preferably 1 to 36 carbon atoms; more preferable examples are optionally substituted saturated straight chain alkyl groups comprising 1 to 20 carbon atoms; and even more preferable examples are such alkyl groups comprising 1 to 3 carbon atoms. As the halogen atom, fluorine, chlorine, bromine, and iodine atoms are preferable; fluorine, chlorine, and bromine atoms are more preferable; and fluorine atom is even more preferable. Specific examples of R₉ and R₁₀ are independently saturated straight chain alkyl groups such as methyl, trifluoromethyl, difluoromethyl, monofluoromethyl, dichloromethyl, mono-chloromethyl, dibromomethyl, difluorochloromethyl, ethyl, pentafluoroethyl, tetrafluoroethyl, trifluoroethyl, tri-fluorochloroethyl, difluoroethyl, monofluoroethyl, tri-fluoroiodoethyl, propyl, heptafluoropropyl, hexafluoropropyl, pentafluoropropyl, tetrafluoropropyl, trifluoropropyl, difluoropropyl, monofluoropropyl, perfluorobutyl, perfluorohexyl, perfluorooctyl, and perfluorooctylethyl; unsaturated alkyl groups such as allyl, tetrafluoroallyl, trifluoroethylene, and perfluorobutylethylene; branched alkyl groups such as isopropyl, pentafluoroisopropyl, hep-tafluoroisopropyl, perfluoro-3-methylbutyl, and perfluo-ro-3-methylhexyl; and cyclic alkyl groups such as cyclohexyl and in general R₉ and R₁₀ are preferably the same. Also, R₉ and R₁₀ may be bonded to each other to form a cyclic alkyl group.

R₉ and R₁₀ are preferably trifluoromethyl, difluoromethyl, monofluoromethyl, pentafluoroethyl, tetrafluoroethyl, trifluoroethyl, difluoroethyl, heptafluoropropyl, hexafluoropropyl, pentafluoropropyl, tetrafluoropropyl, and trifluoropropyl; more preferably trifluoromethyl, difluoromethyl, pentafluoroethyl, trifluoroethyl, heptafluoropropyl, and tetrafluoropropyl; and even more preferably trifluoromethyl. With respect to the above-exemplified respecttive groups, the alkyl group portions are normal (straight chain) unless specified otherwise.

The rings A and B in the general formula (1) may have one to four substituent groups at positions other than 1- and 4-positions. The substituent groups to be bonded may be a halogen atom, hydroxyl, a lower alkoxy group, a cyano group, and a lower alkyl. Examples of the halogen atom are fluorine atom, chlorine atom, bromine atom, and iodine atom. Examples of the alkoxy group are C1 to C5 alkoxy groups such as methoxy and ethoxy and examples of the lower alkyl group are C1 to C5 alkyl such as methyl and ethyl. The rings A and B are preferably unsubstituted or substituted with a halogen atom (particularly chlorine atom, bromine atom, and fluorine atom), methyl, or cyano group.

Additionally, in the case of having a substituent group in each ring B, it is preferable that all four rings B are the same and that the position for the substituent group is m-position in relation to the nitrogen atom bonded to the phenylenediamine skeleton from the standpoint of the preparation. Further, the rings A and B are more preferable to have no substituent group other than the 1- and 4-positions from the standpoint of the preparation.

R₁ to R₈ independently denote hydrogen atom or an optionally substituted aliphatic hydrocarbon group. The aliphatic hydrocarbon group means a group obtained by removing one hydrogen atom from saturated and unsaturated, straight chain, branched, and cyclic hydrocarbons. The number of carbon atoms is preferably 1 to 36 and more preferably 1 to 20. Specific examples are methyl, ethyl, n-propyl, iso-propyl, n-butyl, iso-butyl, sec-butyl, tert-butyl, n-pentyl, iso-amyl (iso-pentyl), tert-pentyl, octyl, decyl, dodecyl, octadecyl, isopropyl, cyclopentyl, cyclohexyl, vinyl, allyl, propenyl, pentenyl, butenyl, hexenyl, hexadienyl, isopropenyl, isohexenyl, cyclohexenyl, cyclopentadienyl, ethynyl, propynyl, hexynyl, isohexynyl, and cyclohexynyl. Among them, more preferable examples are C1 to C5 straight, or branched, saturated aliphatic hydrocarbon groups or unsaturated aliphatic hydrocarbon groups such as methyl, ethyl, n-propyl, iso-propyl, n-butyl, iso-butyl, sec-butyl, tert-butyl, n-pentyl, iso-amyl (iso-pentyl), tert-pentyl, vinyl, allyl, propenyl, and pentenyl. These groups may further be substituted.

As the substituent groups, examples are halogen atoms (e.g. F, Cl, and Br), hydroxyl, alkoxy groups (e.g. methoxy, ethoxy, and isobutoxy), alkoxyalkoxy groups (e.g. methoxyethoxy), aryl (e.g., phenyl and naphthyl and this aryl may further have substituent groups), aryloxy groups (e.g. phenoxy), acyloxy groups (e.g. acetyloxy group, butylyloxy, hexylyloxy, and benzoyloxy group and the aryloxy groups may further have substituent groups), amino groups, alkyl-substituted amino groups (e.g. methylamino and dimethylamino), cyano groups, nitro groups, carboxyl, alkoxycarbonyl (e.g. methoxycarbonyl, and ethoxycarbonyl), amido groups (e.g. acetamido group), sulfonamido group (e.g. methanesulfonamido group), and sulfo groups. Among these substituent groups, a halogen atom, a cyano group, a nitro group, hydroxyl, carboxyl, a carbonylamido group, alkoxycarbonyl, acyl, aryl, and an alkoxy group are preferable.

Preferable examples of the R₁ to R₈ groups are unsubstituted straight chain alkyl groups (of 1 to 6 carbon atoms, more preferably 1 to 3 carbon atoms), unsubstituted branched alkyl groups (particularly branched alkyl groups of 1 to 8 carbon atoms), unsubstituted unsaturated aliphatic hydrocarbon groups (particularly, unsaturated aliphatic hydrocarbon groups of 1 to 8 carbon atoms), cyano-substituted alkyl groups (particularly cyanoalkyl groups of 1 to 8 carbon atoms), alkoxy-substituted alkyl groups (particularly C1 to C3 alkoxy-substituted alkyl groups of 1 to 8 carbon atoms), halogen-substituted alkyl groups (particularly fluorine-substituted alkyl groups of 1 to 8 carbon atoms), and aryl-substituted alkyl groups (particularly phenyl-substituted alkyl groups of 1 to 5 carbon atoms).

More preferable examples are C1 to C8 alkyl groups such as methyl, ethyl, n-propyl, isopropyl, n-butyl, sec-butyl, isobutyl, pentyl, iso-amyl (isopentyl), hexyl, heptyl, and octyl; cyano-substituted C1 to C6 alkyl groups such as cyanomethyl, 2-cyanoethyl, 3-cyanopropyl, 2-cyanopropyl, 4-cyanobutyl, 3-cyanobutyl, 2-cyanobutyl, 5-cyanopentyl, 4-cyanopentyl, 3-cyanopentyl, 2-cyanopentyl, 3,4-dicyanobutyl; alkoxy-substituted C1 to C6 alkyl groups such as methoxyethyl, ethoxyethyl, 3-methoxypropyl, 3-ethoxypropyl, 4-methoxybutyl, 4-ethoxybutyl, 5-ethoxypentyl, and 5-methoxypentyl; and fluorinated C1 to C8 alkyl groups such as trifluoromethyl, monofluoromethyl, pentafluoroethyl, tetrafluoroethyl, trifluoroethyl, heptafluoropropyl, perfluorobutyl, perfluorobutylethyl, perfluorohexyl, perfluorohexylethyl, perfluorooctyl, and perfluorooctylethyl.

These groups may preferably be used by mixture and for example, a mixture of unsubstituted straight chain alkyl groups and cyano-substituted alkyl groups, a mixture of unsubstituted branched chain alkyl groups and cyano-substituted alkyl groups or a mixture of unsubstituted straight chain alkyl groups and unsubstituted branched alkyl groups are preferable.

The compound of the invention defined by the general formula (1) can be prepared in conformity with a method described in, for example, the Patent Document No. 3. That is, a compound defined by the following general formula (4) 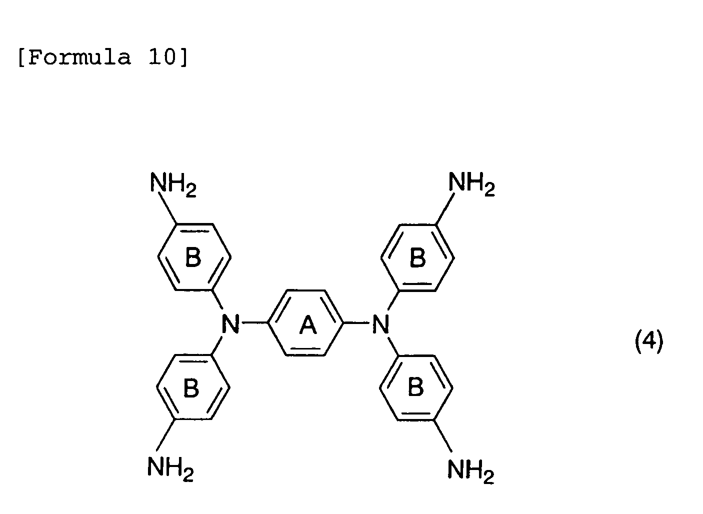 wherein the rings A and B independently denote as described above and obtained by reducing a product obtained by Ullmann reaction of p-phenylenediamine and 1-chloro-4-nitrobenzene is reacted with a halogen compound having a group according to R₁ to R₈ as desired (e.g. n-C₄H₉Br in the case all of R₁ to R₈ are n-C₄H₉) in an organic solvent, preferably a water-soluble polar solvent such as DMF (dimethylformamide), DMI (dimethylimidazolidinone) or NMP (N-methylpyrrolidone) at 30 to 160°C, preferably 50 to 140°C to obtain a compound having the same substituent groups for all of R₁ to R₈ (hereinafter referred to as a full-substituted compound) (the general formula (2)) . Also, in the case of preparing a compound defined by the general formula (2) other than the compound having the same substituent groups for all of R₁ to R₈ (e.g. a precursor of the following compound example No. 34), at first reaction with a reagent (n-C₄H₉Br) in a prescribed molar number (4 mole per 1 mole of a compound defined by the above-mentioned general formula (4)) is carried out to introduce four n-butyl groups into four of the substituent groups R₁ to R₈ and then reaction with another reagent (iso-C₄H₉Br) in a needed molar number (4 mole per 1 mole of a compound defined by the above-mentioned general formula (4)) is carried out to introduce 4 iso-butyl groups into the rest of the substituent groups and consequently obtain the compound defined by the general formula (2) . Any desired compound other than the full-substituted compound can be obtained by a method similar to the exemplified method of preparing the Compound No. 34.

After that, the compound prepared as above and defined by the general formula (2) 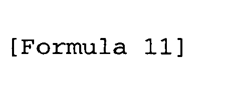 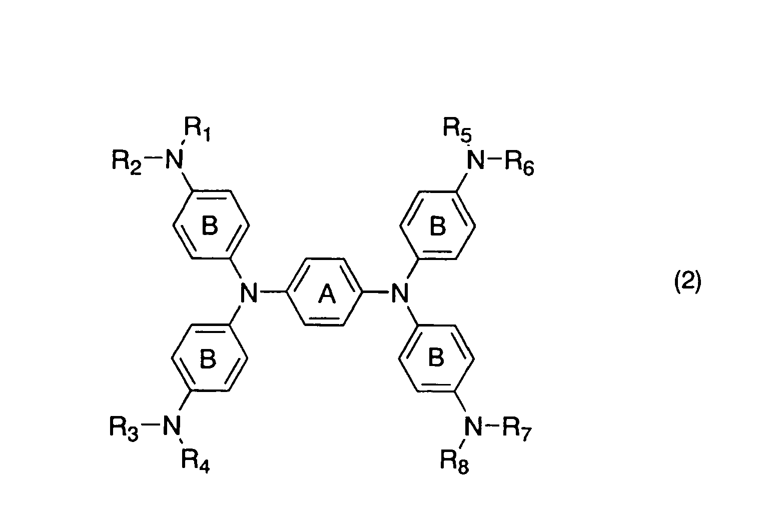 is oxidized by adding 2 equivalent amount of an oxidizing agent (e.g. a silver salt) defined by the following formula (3) in an organic solvent, preferably a water-soluble polar solvent such as DMF, DMI, or NMP at 0 to 100°C, preferably 5 to 70°C. Alternatively, the compound prepared as above and defined by the general formula (2) is oxidized with an oxidizing agent such as silver nitrate, silver perchlorate, or cupric chloride and then an acid or a salt of the anion defined by the general formula (3) is added to the resulting reaction solution. Still alternatively, the compound prepared as above and defined by the general formula (2) is oxidized by adding a silver salt of a mineral acid such as silver nitrate or silver perchlorate and an acid or an alkali metal salt such as a lithium, sodium, or potassium salt of the anion defined by the general formula (3) to obtain the compound defined by the general formula (1).

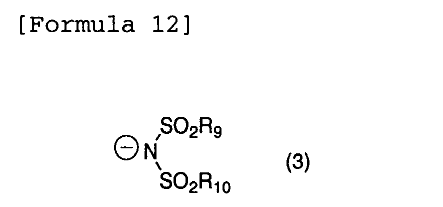

Here, specific examples of the near-IR absorption compounds defined by the general formula (1) of the invention are shown in Table 1. In the Table, with respect to R₁ to R₈, i- means branched state just as "iso-" and PH stands for phenyl. With respect to A and B, in the case there are no substituent groups at the positions other than 1- and 4-positions, they are expressed as 4H and the substitution positions are positions in relation to the nitrogen atoms bonded to the phenylenediamine skeleton structure. Also, with respect to R₁ to R₈, in the case R₁ to R₈ are all butyl, they are expressed as "4(n-C4H9, n-C4H9)" for short and in the case one is iso-pentyl and the rest are n-butyl, that is, in the case one combination among four combinations of the substituent groups contains iso-pentyl and the remaining three combinations are all n-butyl, they are expressed as "3 (n-C4H9, n-C4H9) (n-C4H9, i-C5H11)" for short. Also, in the case that two neighboring R groups bonded to a nitrogen atom are bonded to each other and form a piperidine ring, the formed ring is expressed as "(piperidine ring)". The "cy" means cyclo. In the groups R₉ and R₁₀, the portion of alkyl of 3 or more carbon atoms are all normal (straight chain).

**[Table 1]**

| NO. | (R1, R2) (R3, R4) (R5, R6) (R7, R8) | A | B | R9 | R10 |
|---|---|---|---|---|---|
| 1 | 4 (n-C4H9, n-C4H9) | 4H | 4H | CF3 | CF3 |
| 2 | 4 (i-C4H9, i-C4H9) | 4H | 4H | CF3 | CF3 |
| 3 | 4(CH2CH2CH2CN,CH2CH2CH2CN) | 4H | 4H | CF3 | CF3 |
| 4 | 4(i-C5H11, i-C5H11) | 4H | 4H | CF3 | CF3 |
| 5 | 4(n-C5H11, n-C5H11) | 4H | 4H | CF3 | CF3 |
| 6 | 4(i-C5H11, n-C5H11) | 4H | 4H | CF3 | CF3 |
| 7 | 4(C2H40CH3,C2H40CH3) | 4H | 4H | CF3 | CF3 |
| 8 | 4(CH2CH=CH2,CH2CH=CH2) | 4H | 4H | CF3 | CF3 |
| 9 | 4(CH2CH2CH2CH2CN,CH2CH2CH2CH2CN) | 4H | 4H | CF3 | CF3 |
| 10 | 4(n-C3H7,n-C3H7) | 4H | 4H | CF3 | CF3 |
| 11 | 4(i-C3H7, i-C3H7) | 4H | 4H | CF3 | CF3 |
| 12 | 4(C2H5, C2H5) | 4H | 4H | CF3 | CF3 |
| 13 | 4(CH3, CH3) | 4H | 4H | CF3 | CF3 |
| 14 | 4(n-C3H6COOH, n-C3H6COOH) | 4H | 4H | CF3 | CF3 |
| 15 | 4(CH2PH, CH2PH) | 4H | 4H | CF3 | CF3 |
| 16 | 4 (CF3, CF3) | 4H | 4H | CF3 | CF3 |
| 17 | 4 (CF2CF3, CF2CF3) | 4H | 4H | CF3 | CF3 |
| 18 | 4 (n-C3F7, n-C3F7) | 4H | 4H | CF3 | CF3 |
| 19 | 4(i-C3F7,i-C3F7) | 4H | 4H | CF3 | CF3 |
| 20 | 4(n-C4F9, n-C4F9) | 4H | 4H | CF3 | CF3 |
| 21 | 4(i-C4F9,i-C4F9) | 4H | 4H | CF3 | CF3 |
| 22 | 4(t-C4F9,t-C4F9) | 4H | 4H | CF3 | CF3 |
| 23 | 4(t-C4H9, t-C4H9) | 4H | 4H | CF3 | CF3 |
| 24 | 4(n-C6H13, n-C6H13) | 4H | 4H | CF3 | CF3 |
| 25 | 4(cy-C6H11, cy-C6H11) | 4H | 4H | CF3 | CF3 |
| 26 | 4(cy-C6H10) | 4H | 4H | CF3 | CF3 |
| 27 | 4(C2H4C2F5,C2H4C2F5) | 4H | 4H | CF3 | CF3 |
| 28 | 4 (C2H4C6F13, C2H4C6F13) | 4H | 4H | CF3 | CF3 |
| 29 | 4(C2H4C8F17, C10H21) | 4H | 4H | CF3 | CF3 |
| 30 | 4(C2H40C2H40CH3,C2H40C2H40CH3) | 4H | 4H | CF3 | CF3 |
| 31 | 4 (C2H3 (CH3) C2H5, C2H3 (CH3) C2H5) | 4H | 4H | CF3 | CF3 |
| 32 | 4(s-C4H9,s-C4H9) | 4H | 4H | CF3 | CF3 |
| 33 | 4 (C2H3 (C2H5) 2, C2H3 (C2H5) 2) | 4H | 4H | CF3 | CF3 |
| 34 | 4(n-C4H9,i-C4H9) | 4H | 4H | CF3 | CF3 |
| 35 | 4 (n-C4H9, CH2CH2CH2CN) | 4H | 4H | CF3 | CF3 |
| 36 | 4 (i-C4H9, CH2CH2CH2CN) | 4H | 4H | CF3 | CF3 |
| 37 | 4 (i-C5H11, CH2CH2CH2CN) | 4H | 4H | CF3 | CF3 |
| 38 | 4 (CH2PH, CH3) | 4H | 4H | CF3 | CF3 |
| 39 | 3 (i-C4H9, i-C4H9) (i-C4H9, n-C4H9) | 4H | 4H | CF3 | CF3 |
| 40 | 3 (n-C3H7, n-C3H7) (i-C4H9, n-C3H7) | 4H | 4H | CF3 | CF3 |
| 41 | 3 (i-C5H11, i-C5H11) (i-C5H11, CH2CH2CH2CN) | 4H | 4H | CF3 | CF3 |
| 42 | 2 (n-C4H9, CH2CH2CH2CN) 2 (n-C4H9, i-C4H9) | 4H | 4H | CF3 | CF3 |
| 43 | 3(n-C3H6CN,n-C3H6CN)(n-C3H6CN,n-C4H9) | 4H | 4H | CF3 | CF3 |
| 44 | 4(n-C3H6CN,C2H40CH3) | 4H | 4H | CF3 | CF3 |
| 45 | 4(n-C4H9,C2H40CH3) | 4H | 4H | CF3 | CF3 |
| 46 | 4 (n-C3F7, n-C4H9) | 4H | 4H | CF3 | CF3 |
| 47 | 4(n-C3H6CN,CH2CH=CH2) | 4H | 4H | CF3 | CF3 |
| 48 | 4(n-C4H9, C3H6COOH) | 4H | 4H | CF3 | CF3 |
| 49 | 4 (n-C3H6I, C3H6I) | 4H | 4H | CF3 | CF3 |
| 50 | 4(n-C3H6N02, n-C3H6N02) | 4H | 4H | CF3 | CF3 |
| 51 | 4(n-C4H80H,n-C4H80H) | 4H | 4H | CF3 | CF3 |
| 52 | 4(n-C3H6COOCH3, n-C3H6COOCH3) | 4H | 4H | CF3 | CF3 |
| 53 | 4(n-C3H6CONH2, n-C3H6CONH2) | 4H | 4H | CF3 | CF3 |
| 54 | 4(n-C3H6CONHCH3,n-C3H6CONHCH3) | 4H | 4H | CF3 | CF3 |
| 55 | 4 (n-C3H6CONHPH, n-C3H6CONHPH) | 4H | 4H | CF3 | CF3 |
| 56 | 4(n-C3H6COPH,n-C3H6COPH) | 4H | 4H | CF3 | CF3 |
| 57 | 4(n-C3H6COCH3,n-C3H6COCH3) | 4H | 4H | CF3 | CF3 |
| 58 | 4(C2H4PH,C2H4PH) | 4H | 4H | CF3 | CF3 |
| 59 | 4(CH2PHCH3,CH2PHCH3) | 4H | 4H | CF3 | CF3 |
| 60 | 4(n-C3H6COOK, n-C3H6COOK) | 4H | 4H | CF3 | CF3 |
| 61 | 4(n-C4H9,n-C4H9) | 4H | 4H | C2F5 | C2F5 |
| 62 | 4(i-C4H9,i-C4H9) | 4H | 4H | C2F5 | C2F5 |
| 63 | 4(CH2CH2CH3CN,CH2CH2CH2CN) | 4H | 4H | C2F5 | C2F5 |
| 64 | 4(i-C5H11,i-C5H11) | 4H | 4H | C2F5 | C2F5 |
| 65 | 4(n-C5H11,n-C5H11) | 4H | 4H | C2F5 | C2F5 |
| 66 | 4(i-C5H11,n-C5H11) | 4H | 4H | CF3 | C2F5 |
| 67 | 4(C2H40CH3,C2H40CH3) | 4H | 4H | C2F5 | C2F5 |
| 68 | 4 (CH2CH=CH2, CH2CH=CH2) | 4H | 4H | C2F5 | C2F5 |
| 69 | 4(CH2CH2CH2CH2CN,CH2CH2CH2CH2CN) | 4H | 4H | C2F5 | C2F5 |
| 70 | 4 (n-C3H7, n-C3H7) | 4H | 4H | C2F5 | C2F5 |
| 71 | 4(i-C3H7,i-C3H7) | 4H | 4H | C2F5 | C2F5 |
| 72 | 4 (C2H5, C2H5) | 4H | 4H | C2F5 | C2F5 |
| 73 | 4 (CH3, CH3) | 4H | 4H | C2F5 | C2F5 |
| 74 | 4 (n-C3H6C00H, n-C3H6C00H) | 4H | 4H | C3F7 | C3F7 |
| 75 | 4 (CH2PH, CH2PH) | 4H | 4H | C2F5 | C2F5 |
| 76 | 4 (CF3, CF3) | 4H | 4H | C2F5 | C2F5 |
| 77 | 4(CF2CF3,CF2CF3) | 4H | 4H | CH3 | CH3 |
| 78 | 4(n-C3F7, n-C3F7) | 4H | 4H | C2F5 | C2F5 |
| 79 | 4(i-C3F7, i-C3F7) | 4H | 4H | C2F5 | C2F5 |
| 80 | 4(n-C4F9,n-C4F9) | 4H | 4H | C4F9 | C4F9 |
| 81 | 4 (i-C4F9, i-C4F9) | 4H | 4H | C2F5 | C2F5 |
| 82 | 4(t-C4F9,t-C4F9) | 4H | 4H | C3F7 | C3F7 |
| 83 | 4(t-C4H9, t-C4H9) | 4H | 4H | C2F5 | C2F5 |
| 84 | 4(n-C6H13, n-C6H13) | 4H | 4H | C2F5 | C2F5 |
| 85 | 4(cy-C6H11, cy-C6H11) | 4H | 4H | C2F5 | C2F5 |
| 86 | 4(piperidine ring) | 4H | 4H | C2H5 | C2H5 |
| 87 | 4(C2H4C2F5,C2H4C2F5) | 4H | 4H | C2F5 | C2F5 |
| 88 | 4(C2H4C6F13,C2H4C6F13) | 4H | 4H | C2F5 | C2F5 |
| 89 | 4 (C2H4C8F17,C2H4C8H17) | 4H | 4H | C2F5 | C2F5 |
| 90 | 4(C2H40C2H40CH3,C2H40C2H40CH3) | 4H | 4H | C2F5 | C2F5 |
| 91 | 4 (C2H3 (CH3) C2H5, C2H3 (CH3) C2H5) | 4H | 4H | C3F7 | C3F7 |
| 92 | 4(s-C4H9,s-C4H9) | 4H | 4H | C2F5 | C2F5 |
| 93 | 4 (C2H3 (C2H5) 2, C2H3 (C2H5) 2) | 4H | 4H | (piperidine ring) | |
| 94 | 4(n-C4H9, i-C4H9) | 4H | 4H | C2F5 | C2F5 |
| 95 | 4(n-C4H9,CH2CH2CH2CN) | 4H | 4H | C3F7 | C3F7 |
| 96 | 4 (i-C4H9, CH2CH2CH2CN) | 4H | 4H | C2F5 | C2F5 |
| 97 | 4(i-C5H11, CH2CH2CH2CN) | 4H | 4H | C2F5 | C2F5 |
| 98 | 4(CH2PH, CH3) | 4H | 4H | C2F5 | C2F5 |
| 99 | 3(i-C4H9, i-C4H9)(i-C4H9, n-C4H9) | 4H | 4H | C2H4C6F13C2F5 | |
| 100 | 3 (n-C3H7, n-C3H7) (i-C4H9, n-C3H7) | 4H | 4H | C2F5 | C2F5 |
| 101 | 3(i-C5H11, i-C5H11)(i-C5H11, CH2CH2CH2CN) | 4H | 4H | C3F7 | C3F7 |
| 102 | 2 (n-C4H9, CH2CH2CH2CN) 2(n-C4H9, i-C4H9) | 4H | 4H | C2F5 | C2F5 |
| 103 | 3(n-C3H6CN,n-C3H6CN)(n-C3H6CN,n-C4H9) | 4H | 4H | C2F5 | C2F5 |
| 104 | 4(n-C3H6CN,C2H40CH3) | 4H | 4H | C3F7 | C3F7 |
| 105 | 4 (n-C4H9, C2H40CH3) | 4H | 4H | C8F17 | C8F17 |
| 106 | 4 (n-C3F7, n-C4H9) | 4H | 4H | C2F5 | C2F5 |
| 107 | 4 (n-C3H6CN, CH2CH=CH2) | 4H | 4H | C3F7 | C3F7 |
| 108 | 4 (n-C4H9, C3H6COOH) | 4H | 4H | C2F5 | C2F5 |
| 109 | 4 (n-C3H6I, C3H6I) | 4H | 4H | C3F7 | CF3 |
| 110 | 4(n-C3H6N02, n-C3H6NO2) | 4H | 4H | C3F7 | C3F7 |
| 111 | 4(n-C4H80H,n-C4H80H) | 4H | 4H | C2F5 | C2F5 |
| 112 | 4(n-C3H6COOCH3, n-C3H6COOCH3) | 4H | 4H | C2HF4 | C2HF4 |
| 113 | 4(n-C3H6CONH2,n-C3H6CONH2) | 4H | 4H | C2F5 | C2F5 |
| 114 | 4(n-C3H6CONHCH3,n-C3H6CONHCH3) | 4H | 4H | C3F7 | C3F7 |
| 115 | 4(n-C3H6CONHPH,n-C3H6CONHPH) | 4H | 4H | C4F9 | C4F9 |
| 116 | 4(n-C3H6COPH,n-C3H6COPH) | 4H | 4H | C2F5 | C2F5 |
| 117 | 4 (n-C3H6COCH3, n-C3H6COCH3) | 4H | 4H | CH3 | CH3 |
| 118 | 4(C2H4PH,C2H4PH) | 4H | 4H | C2F5 | C2F5 |
| 119 | 4(CH2PHCH3,CH2PHCH3) | 4H | 4H | C2F5 | C2F5 |
| 120 | 4(n-C3H6COOK,n-C3H6COOK) | 4H | 4H | C2F5 | C2F5 |
| 121 | 4 (n-C4H9, n-C4H9) | o-Cl | 4H | CF3 | CF3 |
| 122 | 4(i-C4H9,i-C4H9) | m-CH3 | 4H | CF3 | CF3 |
| 123 | 4(CH2CH2CH3CN,CH2CH2CH2CN) | 4H | 2-Cl | CF3 | CF3 |
| 124 | 4(i-C5H11,i-C5H11) | 4H | 2-CH3 | CF3 | CF3 |
| 125 | 4(n-C5H11, n-C5H11) | o-Cl | 4H | CF3 | CF3 |
| 126 | 4(i-C5H11, n-C5H11) | o-Br | 4H | CF3 | CF3 |
| 127 | 4 (C2H40CH3, C2H40CH3) | 4H | 2-CN | CF3 | CF3 |
| 128 | 4 (CH2CH=CH2, CH2CH=CH2) | o-C2H5 | 4H | CF3 | CF3 |
| 129 | 4(CH2CH2CH2CH2CN,CH2CH2CH2CH2CN) | o-CH3 | 4H | CF3 | CF3 |
| 130 | 4 (n-C3H7, n-C3H7) | 4H | 2-CH3 | CF3 | CF3 |
| 131 | 4 (i-C3H7, i-C3H7) | 4H | 2-CN | CF3 | CF3 |
| 132 | 4(C2H5,C2H5) | m-CH3 | 4H | CF3 | CF3 |
| 133 | 4 (CH3, CH3) | o, m-2Cl | 4H | CF3 | CF3 |
| 134 | 4(n-C3H6COOH,n-C3H6COOH) | m-OCH3 | 4H | CF3 | CF3 |
| 135 | 4(CH2PH,CH2PH) | 4F | 4H | CF3 | CF3 |
| 136 | 4 (CF3, CF3) | 4F | 4H | CF3 | CF3 |
| 137 | 4(CF2CF3,CF2CF3) | 4H | 3-CN | CF3 | CF3 |
| 138 | 4 (n-C3F7, n-C3F7) | 4H | 3-CH3 | CF3 | CF3 |
| 139 | 4 (i-C3F7, i-C3F7) | o-I | 4H | CF3 | CF3 |
| 140 | 4 (n-C4F9, n-C4F9) | o-Br | 4H | CF3 | CF3 |
| 141 | 4(i-C4F9,i-C4F9) | o-OH | 4H | CF3 | CF3 |
| 142 | 4 (t-C4F9, t-C4F9) | 4H | 2-OH | CF3 | CF3 |
| 143 | 4 (t-C4H9, t-C4H9) | o-N02 | 4H | CF3 | CF3 |
| 144 | 4(n-C6H13,n-C6H13) | o-OCH3 | 4H | CF3 | CF3 |
| 145 | 4(cy-C6H11,cy-C6H11) | o-F | 4H | CF3 | CF3 |
| 146 | 4(piperidine ring) | o,m-2F | 4H | CF3 | CF3 |
| 147 | 4(C2H4C2F5,C2H4C2F5) | o-C2H5 | 4H | CF3 | CF3 |
| 148 | 4(C2H4C6F13,C2H4C6F13) | o-C2H5 | 4H | CF3 | CF3 |
| 149 | 4 (C2H4C8F17, C2H4C8H17) | 4H | 2, 5-2CN | CF3 | CF3 |
| 150 | 4(C2H40C2H40CH3,C2H40C2H40CH3) | 4H | 2,4-2CH3 | CF3 | CF3 |

A resin composition of the invention contains a diimmonium compound of the invention in a resin.

Specific examples of the resin to be used are vinyl compounds and addition polymers of vinyl compounds such as polyethylene, polystyrene, poly(acrylic acid), poly(acrylic acid ester), poly(vinyl acetate), polyacrylonitrile, poly(vinyl chloride), and poly(vinyl fluoride); poly(methacrylic acid), poly(methacrylic acid ester), poly(vinylidene chloride), poly (vinylidene fluoride), poly (vinylidene cyanide); copolymers of vinyl compounds or fluoro compounds such as vinylidene fluoride- trifluoroethylene copolymer, vinylidene fluoride-tetrafluoroethylene copolymer, and vinylidene cyanide-vinyl acetate copolymer; fluorine-containing resins such as polytrifluoroethylene, polytetrafluoroethylene, and polyhexafluoropropylene; polyamides such as nylon 6 and nylon 66; polyimides; polyurethanes; polypeptides; polyesters such as polyethylene terephthalate; polycarbonate; polyethers such as polyoxymethylene; epoxy resins; polyvinyl alcohol; and polyvinyl butyral.

The method of producing the resin composition of the invention is not particularly limited and for example, the following well-known methods can be employed. For example, (1) the diimmonium compound of the invention is kneaded with a resin, heated and formed to produce a resin plate or a film; (2) the resin monomers or prepolymers of resin monomers together with the diimmonium compound of the invention is cast-polymerized in the presence of a polymerization catalyst to produce a resin plate or a film; (3) a coating composition containing the diimmonium compound of the invention is prepared and applied to a transparent resin plate, a transparent film or a transparent glass plate; and (4) the diimmonium compound of the invention is added to an adhesive and used to produce a laminated resin plate, a laminated resin film, or a laminated glass plate.

In the above-mentioned production method (1), although the processing temperatures and film formation (or resin plate formation) conditions differ slightly depending on the resin to be used, generally, the diimmonium compound of the invention is added to a powder or a pellet of a base resin and heated at 150 to 350°C for melting and then the melt is formed to produce a resin plate or formed into a film (or a resin plate) by an extruder. Although the amount of addition of the diimmonium compound of the invention differs depending on the thickness, the absorption intensity, and visible light transmittance of the resin plate or film to be produced, generally, it is 0.01 to 30% by weight, preferably 0.03 to 15% by weight based on the weight of the binder resin.

In the above-mentioned method (2) for the production by cast-polymerizing the resin monomers or prepolymers of resin monomers together with the above-mentioned compound in the presence of a polymerization catalyst, the mixture is injectted into a mold and cured by reaction or injected into a die and hardened for forming to obtain a hard product. Many resins are formable in this process and specific examples of such resins are acrylic resin, diethylene glycol bis(allylcarbonate) resin, epoxy resin, phenol-formaldehyde resin, polystyrene resin, silicone resin, and the like. Among them, a casting method based on bulk polymerization of methyl methacrylate from which an acrylic sheet excellent in hardness, heat resistance, and chemical resistance is preferable.

As the polymerization catalyst, known thermal radical polymerization initiators are usable and peroxides such as benzoyl peroxide, p-chlorobenzoyl peroxide, and diisopropyl peroxycarbonate and azo compounds such as azobisisobutyronitrile can be exemplified. The amount to be used is generally 0.01 to 5% by weight based on the total weight of the mixture. The temperature of heating in the thermal polymerization is generally 40 to 200°C and the polymerization time is generally about 30 minutes to 8 hours. Other than the thermal polymerizetion, a photopolymerization method by adding a photopolymerization initiator and a sensitizer can be employed.

As the above-mentioned method (3), a method by dissolving the diimmonium compound of the invention in a binder resin and an organic solvent to obtain a coating composition and a method by finely pulverizing the compound and dispersing the fine particles to obtain a water-based coating composition are available. In the former method, for example, aliphatic ester resin, acrylic resin, melamine resin, urethane resin, aromatic ester resin, polycarbonate resin, polyvinyl type resin, aliphatic polyolefin resin, aromatic polyolefin resin, polyvinyl alcohol resin, polyvinyl-modified resin, or their copolymer resin may be used as the binder.

As the solvent, halogen type, alcohol type, ketone type, ester type, aliphatic hydrocarbon type, aromatic hydrocarbon type, and ether type solvents and their mixtures may be used. Although the concentration of the diimmonium compound of the invention differs depending on the thickness, the absorption intensity, and visible light transmittance of the coating to be formed, generally, it is 0.1 to 30% by weight.

The coating composition produced in such a manner is applied to a transparent resin film, a transparent plate or a transparent glass plate by a spin coater, a bar coater, a roll coater, or a spray to obtain a near-IR absorption filter.

In the above-mentioned method (4), as the adhesive, known transparent adhesives for general resins such as silicone type, urethane type, or acrylic type adhesives and adhesives for laminated glass such as a polyvinyl butyral adhesive and ethylene-vinyl acetate type adhesive can be used. With an adhesive containing 0.1 to 30% by weight of the diimmonium compound of the invention, a filter is produced by sticking together two transparent resin plates; a resin plate and a resin film; a resin plate and glass, two resin films; a resin film and glass, and two glass plates.

In addition, at the time of kneading and mixing in the respective methods, common additives such as a UV absorber, a plasticizer and the like to be used for resin formation may be added.

A near-IR absorption filter of the invention will be described below. The filter may comprise a substrate and a layer containing the diimmonium compound of the invention formed on the substrate or the substrate itself may be a layer containing a resin composition (or its hardened product) containing the near-IR absorption compound. In general, the substrate is not particularly limited if it is usable for near-IR absorption filters. However, substrates made of resins are commonly used. The thickness of the layer containning the near-IR absorption compound is about 0.1 µm to 10 mm and properly determined based on the purposes such as near-IR ray cut (or reduction) ratio. The content of the near-IR absorption compound is also properly determined based on the aimed near-IR ray cut ratio. Examples of the resins to be used are resins similar to those resin compositions exemplified above and in the case of forming a resin plate or a resin film, those having transparency as high as possible are preferable. Examples of the method for producing the near-IR absorption filter are methods similar to those described for the production of the above-mentioned resin compositions.

As an IR absorption compound of the IR absorption filter of the invention, only one type of diimmonium compound of the invention may be added. Also, two or more types of diimmonium compounds of the invention may be used in combination, and also these diimmonium compounds and other types of near-IR absorption compounds may be used together. Examples of other types of near-IR absorption compounds to be used together are phthalocyanine type dyes, cyanine type dyes, and dithiol-nickel complexes. Examples of usable near-IR absorption compounds of inorganic metal type are metal copper, copper compounds such as copper sulfide and copper oxide, metal mixtures containing zinc oxide as a main component, tungsten compounds, ITO (indium tin oxide), and ATO (antimony tin oxide).

Further, in order to change the color tone of the filter, dyes having absorption in the visible light region (dyes for toning) may be added to an extent that no effect of the inventtion is inhibited. Also, it is possible to produce a filter containing only a dye for toning and then to stick to it a near-IR absorption filter of the invention.

In the case such a near-IR absorption filter is used for a front panel of a plasma display, the higher the transmittance of visible light rays the better, and the transmittance is required to be 40% or higher, preferably 50% or higher. The near-IR ray cut region is preferably 750 to 1,200 nm and more preferably 800 to 1, 000 nm and the average transmittance of the near-IR rays in the region is preferably 50% or lower, more preferably 30% or lower, furthermore preferably 20% or lower, and even more preferably 10% or lower.

The use of the near-IR absorption filter of the invent-tion is not limited only to the front panel of displays and may be used for filters and films for which IR rays have to be cut, such as heat insulation films, optical products, and sunglasses.

The near-IR absorption filter of the invention has a very high transmittance in the visible light region, is free from antimony or arsenic and environment-friendly, and absorbs near-IR rays in a wide region and thus the near-IR absorption filter of the invention is an excellent near-IR absorption filter. As compared with conventional near-IR absorption filters containing no antimony and comprising perchlorate ion, hexaflurophosphate ion, or borofluoride ion, the near-IR absorption filter of the invention is excellent in the stability. Further, the solubility is sufficiently high and the processibility is also excellent. Particularly, the near-IR absorption filter of the invention is remarkably excellent in heat resistance, moisture and heat resistance, and light fastness and is hardly decomposed by heat, so that the near-IR absorption filter scarcely cause coloration in the visible light region. Further owing to such characteristics, it is preferably used for the near-IR absorption filter and the near-IR absorption films such as heat insulation films and sunglasses and particularly preferably for a near-IR absorption filter for a plasma display.

Next an optical information recording medium of the invention will be described.

The optical information recording medium of the inventtion comprises a recording layer on a substrate and the recording layer is characterized in that the diimmonium compound of the invention is contained in the layer. The recording layer may comprise only the diimmonium compound or the diimmonium compound together with various additives such as a binder. In this case, the information is recorded by the diimmonium compound.

Also, a mixture of the diimmonium compounds of the invention may be added to a recording layer of an optical information recording medium in which the information is recorded by an organic dye, so that the light fastness of the optical information recording medium can be improved. Such an optical information recording medium is also included in the optical information recording medium of the invention.

Examples of organic dyes to be used in combination with the diimmonium compound of the invention in the optical information recording medium are generally known dyes, e.g. cyanine type dyes, squarylium type dyes, indoaniline type dyes, phthalocyanine type dyes, azo type dyes, merocyanine type dyes, polymethine type dyes, naphthoquinone type dyes, and pyrylium type dyes. Among these organic dyes to be used in combination, cyanine type dyes, squarylium type dyes, indoaniline type dyes, and polymethine type dyes are particularly preferable.

The mixture of the diimmonium compound is generally used in an amount of 0.01 to 10 mols, preferably 0.03 to 3 mols per 1 mol of the organic dyes.

The optical information recording medium of the inventtion comprises a recording layer containing the diimmonium compound of the invention and dyes if desired on a substrate and if necessary, a reflection layer and a protection layer may be formed. As the substrate, any known substrates may be used. For example, a glass plate, a metal plate, or a plastic plate or film may be used. The plastics for producing them are, for example, acrylic resin, polycarbonate resin, methacrylic resin, polysulfone resin, polyimide resin, amorphous polyolefin resin, polyester resin, and polypropylene resin. With respect to the form of the substrate, various forms such as a disk, a card, a sheet, and a roll film-like forms are possible.

To make tracking easy at the time of recording, a guide groove may be formed on the glass or plastic substrate. Also, an undercoating of a plastic binder, an inorganic oxide or an inorganic sulfide may be formed on the glass or plastic substrate and it is preferable that the undercoating has a thermal conductivity lower than that of the substrate.

The recording layer of the optical information recording medium of the invention is formed by dissolving the diimmonium compound of the invention, preferably the diimmonium compound of the invention and other organic dyes in a known organic solvent such as tetrafluoropropanol (TFP), octafluoropentanol (OFP), diacetone alcohol, methanol, ethanol, butanol, methyl cellosolve, ethyl cellosolve, dichloroethane, isophorone, and cyclohexanone; adding a binder if needed; and applying the thus obtained solution to the substrate by a spin coater, a bar coater, or a roll coater. As another method, a vacuum evaporation method, a sputtering method, a doctor blade method, a cast method, or a dipping method in which the substrate is dipped in the solution can be employed to produce the layer. Here, as a binder, acrylic resin, urethane resin, or epoxy resin can be used.

The thickness of the recording layer is preferably 0.01 to 5 µm, more preferably 0.02 to 3 µm, in consideration of the recording sensitivity and reflectance.

If necessary, the optical information recording medium of the invention may have an undercoating layer under the recording layer and a protection layer on the recording layer and further a reflection layer may be formed between the recording layer and the protection layer. In the case of forming a reflection layer, the reflection layer may be of gold, silver, copper or aluminum, preferably of gold, silver or aluminum. These metals may be used alone or as alloys of two or more of the metals. The layer may be formed by a vacuum evaporation method, a sputtering method, or an ion plating method. The thickness of such a reflection layer is 0.02 to 2 µm. The protection layer to be formed on the reflection layer in some cases is generally formed by applying a UV curable resin by a spin coating method and then curing the resin by UV irradiation. In addition, epoxy resin, acrylic resin, silicone resin, and urethane resin may be used as protection layer formation materials. The thickness of such a protection layer is generally 0.01 to 100 µm.

Recording of information or formation of images with the optical information recording medium of the invention is carried out by radiating a converged, spot type high energy beam of laser, e.g. semiconductor laser, helium-neon laser, He-Cd laser, YAG laser, and Ar laser through the substrate or to the recording layer from the side opposite to the substrate, and reading out of the information or the images may be carried out by detecting the difference of the reflection light quantity or transmitted light quantity in pit portions and the positions where no pit is formed by radiating low output laser beam.

The diimmonium compound of the invention has the maximum absorption wavelength in a zone of 900 nm or longer and an absorption peak with a molar absorption coefficient of as high as several ten thousands to over one hundred thousands. Also, from stability tests for heat resistance, light fastness, and moisture-and-heat resistance, the compound is found to get scarcely discolored and excellent in stability as compared with conventional compounds and also from a solubility test, the compound is found to have sufficient solvent-solubility and thus can be used as an IR absorber with good processibility.

The composition of the invention, particularly, the IR absorption filter, has high solubility and excellent processibility and is further excellent in stability such as heat resistance, moisture-and-heat resistance, and light fastness, as compared with near-IR absorption filters containing conventional diimmonium compounds. Particularly, in stability tests for these properties, the near-IR absorption filter of the invention scarcely causes decomposition reaction or coloration in the visible region and thus the near-IR absorption filter is excellent in heat resistance, moisture-and-heat resistance, and light fastness (or stability). Owing to these properties, the composition of the invention can be used as the near-IR absorption filter and the near-IR absorption film for heat insulation films and sunglasses, and particularly useful for the near-IR absorption filter for plasma display.

The optical information recording medium of the invention is provided with remarkably improved light stability by the addition of the compound having the general formula (1), as compared with optical information recording media containing conventional diimmonium compounds. Particularly, the diimmonium compound of the invention has sufficient solubility and excellent in processibility. Further, in the case the diimmonium compound is added to an organic dye thin film, which is a recording layer of an optical information recording medium, the optical information recording medium having remarkably improved durability to repeated regeneration and light stability can be obtained.

### (Examples)

The following examples are presented to better illustrate the invention, but are not to be construed as limiting the invention to the specific embodiments disclosed. "Part" and "%" in the Examples are on the basis of weight unless specified otherwise.

### Example 1

### (Preparation Example 1)

### (Preparation of the Compound No. 1 in Table 1)

3 part of N,N,N',N'-tetrakis[p-di(n-butyl)aminophenyl] -p-phenylenediamine was added to 16.5 part of DMF and dissolved by heating at 60°C and then 1.16 part of silver nitrate and 2.19 part of bis(trifluoromethanesulfonic)imide potassium salt dissolved in 16.5 part of DMF were added to the obtained solution and heated and stirred for 30 minutes. After the insoluble matters were separated by filtration, water was added to the reaction solution and the precipitated crystal was filtered, washed with water, and dried to obtain 4.3 part of the aimed Compound No. 1.
λmax: 1,102 nm (in dichloromethane);
the melting point: around 170°C; and the thermal decomposition point (the weight decrease starting point): around 280°C (measured by TG-DTA)

### Example 2

### (Preparation Example 2)

### (Preparation of the Compound No. 2 in Table 1)

4.3 part of the Compound No. 2 was obtained in the same manner as in Example 1, except that N,N,N',N'-tetrakis [p-di(iso-butyl)aminophenyl]-p-phenylenediamine was used in place of N,N,N',N'-tetrakis[p-di(n-butyl)aminophenyl]-p-phenylenediamine.
λmax: 1,104 nm (in dichloromethane);
the melting point: around 165°C; and the thermal decomposition point (the weight decrease starting point): around 282°C (measured by TG-DTA)

### Example 3

### (Preparation Example 3)

### (Preparation of the Compound No. 3 in Table 1)

3.28 part of N,N,N',N'-tetrakis[p-di(cyanopropyl) aminophenyl]-p-phenylenediamine and 16.5 part of DMF were added to a solution obtained by dissolving 0.58 part of sodium nitrate in 3 part of water. The obtained reaction solution was heated to 60°C and then 1.16 part of silver nitrate dissolved in 16.5 part of DMF was added to the resulting reaction solution and stirred for 30 minutes. After the insoluble matters were separated by filtration, 2.19 part of bis(trifluoromethanesulfonic)imide potassium salt was added to the reaction solution and stirred for 3 hours and water was added. The precipitated crystal was filtered, washed with water, and dried to obtain 4.5 part of the aimed Compound No. 3.
λmax: 1,064 nm (in dichloromethane);
the melting point: around 180°C; and the thermal decomposition point (the weight decrease starting point): around 282°C (measured by TG-DTA)

### Example 4

### (Preparation Example 4)

### (Preparation of the Compound No. 4 in Table 1)

3.7 part of the Compound No. 4 was obtained in the same manner as in Example 1, except that N,N,N',N'-tetrakis [p-di(iso-amyl)aminophenyl]-p-phenylenediamine was used in place of N,N,N',N'-tetrakis[p-di(n-butyl)aminophenyl]-p-phenylenediamine.
λmax: 1,102 nm (in dichloromethane);
the melting point: around 175°C; and the thermal decomposition point (the weight decrease starting point): around 280°C (measured by TG-DTA)

### Example 5

### (Preparation Example 5)

### (Preparation of the Compound No. 9 in Table 1)

4.1 part of the Compound No. 9 was obtained in the same manner as in Example 1, except that N,N,N',N'-tetrakis [p-di(cyanobutyl)aminophenyl]-p-phenylenediamine was used in place of N,N,N',N'-tetrakis[p-di(n-butyl)aminophenyl] -p-phenylenediamine.
λmax: 1,086 nm (in dichloromethane);
the melting point: around 145°C; and the thermal decomposition point (the weight decrease starting point): around 277°C (measured by TG-DTA)

### Example 6

### (Preparation Example 6)

### (Preparation of the Compound No. 12 in Table 1)

2.1 part of the Compound No. 12 was obtained in the same manner as in Example 1, except that N,N,N',N'-tetrakis[p-diethylaminophenyl]-p-phenylenediamine was used in place of N,N,N',N'-tetrakis[p-di(n-butyl)aminophenyl]-p-phenylenediamine.
λmax: 1,084 nm (in dichloromethane);
the melting point: around 186°C; and the thermal decomposition point (the weight decrease starting point): around 278°C (measured by TG-DTA)

### Example 7

### (Preparation Example 7)

### (Preparation of the Compound No. 35 in Table 1)

2.6 part of the Compound No. 35 was obtained in the same manner as in Example 1, except that a mixture of n-butyl derivative and 3-cyanopropyl derivative of N,N,N',N'- tetrakis(p-aminophenyl)-p-phenylenediamine was used in place of N,N,N',N'-tetrakis[p-di(n-butyl)aminophenyl]-p-phenylenediamine.
λmax: 1,090 nm (dichloromethane);
the melting point: around 135°C; and the thermal decomposition point (the weight decrease starting point): around 256°C (measured by TG-DTA)

With respect to Examples of other compounds, they could be prepared by oxidizing corresponding phenylenediamine derivatives with an oxidizing agent and then reacted with corresponding anions similarly to the above-mentioned Preparation Examples 1 to 7.

### Example 8

The compounds obtained in the above-mentioned Examples were subjected to the measurement of molar absorption coefficient (ε) in dichloromethane. The results are shown in Table 2.

### (Comparative Examples 1 and 2)

The molar absorption coefficient (ε) was measured in dichloromethane in the same manner, except that the compounds described in Patent Document No. 2: 1,5-naphthalenedisulfonic acid salt of N,N,N',N'-tetrakis[p-di(n-butyl)aminophenyl] phenylenediimmonium (the compound described in Example 1 of Patent Document No. 2) (Comparative Example 1: Compound No. 151) and 1-hydroxy-2,5-naphthalenedisulfonic acid salt of N,N,N',N'-tetrakis[p-di(n-butyl)aminophenyl] phenylenediimmonium (Comparative Example 2: Compound No. 152) were used. The results are shown in Table 2.

**[Table 2] Table 2 (Comparative test of molar absorption coefficient measurement)**

| Compound No. | molar absorption coefficient (ε) |
|---|---|
| No. 1 | 108,000 |
| No. 2 | 109,000 |
| No. 3 | 109,000 |
| No. 4 | 110,000 |
| No. 9 | 109,000 |
| No. 12 | 96,000 |
| No. 151 (Comparative Example 1) | 82,000 |
| No. 152 (Comparative Example 2) | 24,500 |

As is made clear from the above results, the molar absorption coefficient of the diimmonium compounds of the invention was quite high at 96,000 or higher.

### Example 9

### (Solubility of Diimmonium compounds)

The solubility was measured for the compounds obtained in the above-mentioned Examples at a room temperature in methyl ethyl ketone (MEK) and toluene. The results are shown in Table 3.

### (Comparative Examples 3 and 4)

The solubility was measured in the same manner, except that the compounds described in Patent Document No.3; hexafluoroantimonate of N,N,N',N'-tetrakis[p-di(n-butyl) aminophenyl]phenylenediimmonium (Comparative Example 3: Compound No. 153) and hexafluoroantimonate of N,N,N',N'-tetrakis[p-di(3-cyanopropyl)aminophenyl]phenylenediimmonium (Comparative Example 4: Compound No. 154) were used. The results are shown in Table 3.

### [Table 3]

**Table 3 (Comparative test of solubility measurement)**

| Compound No. | MEK | toluene |
|---|---|---|
| No. 1 | 20% | 0.2% |
| No. 2 | 5% | 0.04% |
| No. 3 | 2% | insoluble |
| No. 4 | 10% | 0.1% |
| No. 153 (Comparative Example 3) | 4.5% | insoluble |
| No. 154 (Comparative Example 4) | insoluble | insoluble |

As is made clear from the above results, as compared with derivatives having similar substituent groups, the diimmonium compounds of the invention had improved solubility in solvents to be used commonly, such as MEK and toluene.

### Example 10

### (Near-IR absorption filter and stability test of moisture-and-heat resistance)

1.2 part of each compound obtained in each Example was dissolved in 18.8 part of MEK. 80 part of a resin solution obtained by dissolving 25 part of an acrylic resin (Dianal BR-80, manufactured by Mitsubishi Rayon Co., Ltd.) in 75 part of MEK was mixed with the obtained solution to obtain a solution for coating. The solution was applied to form a film with a thickness of 2 to 4 *µ*m on a polyester film and dried at 80°C to obtain a near-IR absorption filter of the invention.

The obtained near-IR absorption filter was subjected to a stability test of moisture-and-heat resistance for 14 days in a constant temperature and constant humidity apparatus under 60°C and 95% RH conditions. The filter was subjected to color measurement by a spectrophotometer before and after the test to calculate L*, a*, and b* values. If the + value of b* value is high, it means that the hue is yellowish and if b* value is close to 0, it means that the yellowish degree is low and filter is desirable and therefore, the hue evaluation and stability evaluation were done based on the b* value and its change. The obtained results of the heat resistance test are shown in Table 4.

### (Comparative Examples 5 and 6)

Filters were produced and evaluated in the same manner as in Example 10, except that the compounds described in Patent Document No. 1: hexafluorophosphate of N,N,N',N'-tetrakis [p-di(n-butyl)aminophenyl]-p-phenylenediimmonium (Comparative Example 5: Compound No. 155) and borofluoride salt of N,N,N',N'-tetrakis[p-di(n-butyl)aminophenyl]phenylene diimmonium (Comparative Example 6: Compound No. 156) were used in place of the above-mentioned compounds. The results are shown in Table 4.

### [Table 4]

**Table 4 (Stability test of moisture-and-heat resistance)**

| Compound No. | b* value | | |
|---|---|---|---|
| | initial period | 14-day after | difference |
| No. 1 | 3.5 | 5.3 | 1.8 |
| No. 2 | 2.2 | 4.1 | 1.9 |
| No. 3 | 3.6 | 6.1 | 2.5 |
| No. 4 | 2.2 | 4.3 | 2.1 |
| No. 9 | 2.4 | 4.3 | 1.9 |
| No. 155 (Comparative Example 5) | 2.9 | 9.0 | 6.1 |
| No. 156 (Comparative Example 6) | 3.5 | 14.3 | 10.8 |

Since the near-IR absorption filters containing these compounds of the invention had smaller change in b* values than those of the samples of Comparative Examples, they were found excellent in the stability under high temperature and high humidity. Further, the filters containing those compounds having alkyl groups branched at terminals for all R₁ to R₈ in the above-mentioned general formula (1) had lower b* values from the initial period through 14-day after and therefore they were found having low yellowish degree and excellent as near-IR absorption filters.

### Example 11

### (Example of Optical Information Recording Medium)

0.02 part of the Compound No. 1 obtained in the above-mentioned Preparation Example 1 and 0.10 part of a cyanine dye (OM-57, manufactured by Fuji Photo Film Co., Ltd.) were dissolved in 10 part of tetrafluoropropanol and filtered by a 0.2 µm filter to obtain a coating solution. 1 ml of the solution was dropped on a 5-inch grooved polycarbonate resin substrate by a pipette, applied by a spin coater, and dried to obtain an organic thin film recording layer. The maximum absorption wavelength of the coating film was 719 nm. As a reflection layer, a gold film was formed by sputtering on the obtained coating film to obtain an optical information recording medium. The obtained optical information recording medium was evaluated by a recording regeneration apparatus for CD-R to find that recording and regeneration was possible.

In such a manner, an optical information recording medium excellent in processibility could be obtained and recording and regeneration was carried out without any problem.

### Example 12

### (Stability test of light fastness of cyanine dye film)

0.3 part of the cyanine dye (OM-57) was dissolved in 15 part of tetrafluoropropanol and 0. 04 part of the Compound No. 3 was added to the obtained solution to produce a coating solution. The obtained coating solution was applied to a polycarbonate substrate by spin coating to form a dye film. The obtained dye film was subjected to a light stability test by radiating light for 50 hours from the substrate side in the conditions: light source output: 0.36W/m²; bath temperature: 24°C; black panel temperature: 40°C; humidity: 30% RH: by a Weatherometer (Ci4000, manufactured by Atlas Co.) . After that, the remaining ratio of the cyanine dye was measured by a spectrophotometer. The result is shown in Table 5.

### (Comparative Example 7)

For comparison, a dye film was formed and evaluated in the same manner, except that the compound described in Patent Document No. 3; hexafluoroantimonate of tetrakis [p-di (n-butyl)aminophenyl]phenylenediimmonium (Comparative Example 7: Compound No. 153); was used in place of the Compound No. 1. The result is shown in Table 5.

### [Table 5]

**Table 5 (Stability test of light fastness of cyanine dye film)**

| Compound No. | remaining ratio of cyanine dye (%) | | |
|---|---|---|---|
| | initial period | 100-hour after | 150-hour after |
| No. 3 | 100 | 47 | 27 |
| No. 153 (Comparative Example 7) | 100 | 27 | 0 |

As is made clear from the results, the light stability of the cyanine dye could be improved greatly by the addition of the compound of the invention.

### Example 13

### (Stability test of light fastness of diimmonium compound thin film)

0.1 part of the Compound No. 3 was added to 10 part of tetrafluoropropanol to produce a coating solution. The obtained coating solution was applied to a polycarbonate substrate by spin coating to form a thin film of the diimmonium compound. The obtained thin film was subjected to a light stability test by radiating light from the substrate side for 50 hours in the conditions: light source output: 0.36 W/m²; bath temperature: 24°C; black panel temperature: 40°C; humidity: 30% RH: by a Weatherometer (Ci4000, manufactured by Atlas Co.). After that, the remaining ratio of the diimmonium compound was measured by a spectrophotometer. The result is shown in Table 6.

### (Comparative Example 8)

For comparison, a dye film was formed and evaluated in the same manner, except that the compound described in Patent Document No. 3; hexafluoroantimonate of tetrakis [p-di (n-butyl)aminophenyl]phenylenediimmonium (Comparative Example 8: Compound No. 153) ; was used in place of the Compound No. 3. The result is shown in Table 6.

### [Table 6]

**Table 6 (Stability test of light fastness of diimmonium compound thin film)**

| Compound No. | remaining ratio of diimmonium compound (%) | | |
|---|---|---|---|
| | initial period | 100-hour after | 150-hour after |
| No. 3 | 100 | 88 | 86 |
| No. 153 (Comparative Example 8) | 100 | 81 | 76 |

As is made clear from the results, the compound of the invention is excellent in the light fastness in the case of being in form of a thin film.

### Example 14

### (Near-IR absorption filter and stability test of heat resistance)

Filters were produced in the same manner as in Example 10 and the obtained near-IR absorption filters were kept in an oven at 80°C for 21 days. After that, the filters were subjected to color measurement by a spectrophotometer to calculate L*, a*, and b* values and their stability was evaluated based on the change in b* values. If the b* is low, that is, the absorption in visible ray region is low, the near-IR absorption filter is preferable. The obtained results of the heat resistance test are shown in Table 7.

### (Comparative Example 9)

For comparison, a filter was produced and evaluated in the same manner as in Example 10, except that hexafluoroantimonate of N,N,N',N'-tetrakis[p-di(n-butyl) aminophenyl]phenylenediimmonium (Comparative Example 9: Compound No. 153); was used in place of the above-mentioned compounds. The result is shown in Table 7.

### [Table 7]

**Table 7 (Stability test of heat resistance)**

| Compound No. | b* value | | |
|---|---|---|---|
| | initial period | 14-day after | difference |
| No. 1 | 3.5 | 5.3 | 1.8 |
| No. 2 | 2.2 | 3.7 | 1.5 |
| No. 4 | 2.4 | 3.8 | 1.6 |
| No. 153 (Comparative Example 9) | 2.5 | 4.4 | 1.9 |

Since the near-IR absorption filters of the invention had smaller change in b* values than that of the Comparative Example, it was found that they were excellent in stability in high temperature condition. Among them, especially, the filters with the compound having alkyl groups branched at terminals for all R₁ to R₈ in the diimmonium compound defined by the above-mentioned general formula (1) showed smaller change in b* value change as compared with those having straight chain alkyl groups and therefore it can be understood that the filter is excellent as a near-IR absorption filter. Industrial Applicability

A near-IR absorption compound of the invention is free from antimony and arsenic and therefore is not a toxic substance, has a molar absorption coefficient as high as 90, 000 or higher, and is a compound excellent in heat resistance, light fastness, and solubility. Also, as compared with conventional antimony-free diimmonium compounds containing hexafluorophosphate ion, perchlorate ion, and borofluoride ion, the compound is particularly excellent in heat resistance and moisture-and-heat resistance. A near-IR absorption filter using the compound is free from antimony and remarkably excellent in heat resistance, hardly causes decomposition reaction by heat and is scarcely colorized in the visible ray region. Having such characteristics, the near-IR absorption compound of the invention is preferable to be used for a near-IR absorption filter and a near-IR absorption film for heat insulation films and sunglasses and particularly preferable to be used for a near-IR absorption filter for plasma display. An optical information recording medium of the invention has remarkably improved light fastness as compared with an optical information recording medium comprising a conventional diimmonium compound. The compound of the invention has a sufficiently high solubility and is excellent in processibility. In the case the compound is added to an organic dye thin film, which is a recording layer of an optical information recording medium, the optical information recording medium is provided with greatly improved durability and light fastness stability for repeated regeneration.

## Claims

1. A diimmonium compound having a structure defined by the following general formula (1); 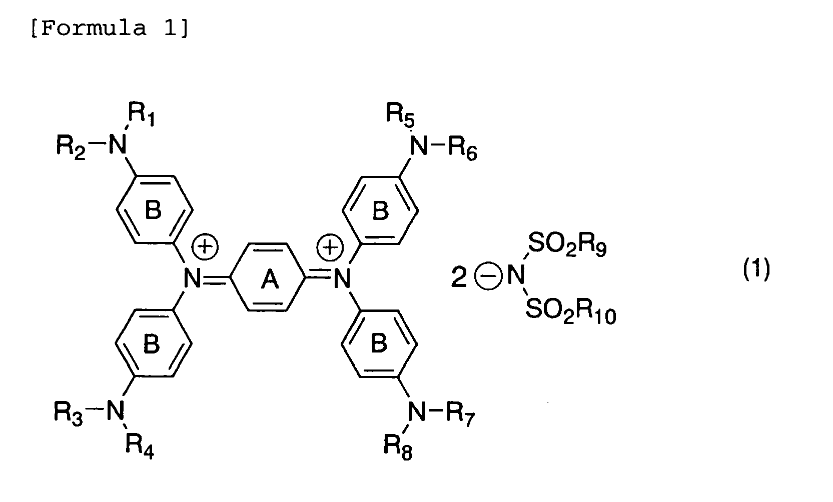 wherein R₁ to R₈ independently denote hydrogen atom or an optionally substituted aliphatic hydrocarbon group; R₉ and R₁₀ independently denote an aliphatic hydrocarbon group optionally containing a halogen atom; and rings A and B may further have substituent groups.

2. A diimmonium compound according to Claim 1, wherein the substituent groups of the optionally substituted aliphatic hydrocarbon groups denoted as R₁ to R₈ of the general formula (1) are independently a halogen atom, a cyano group, a nitro group, a hydroxyl group, a carboxyl group, a carboxyamido group, an alkoxycarbonyl group, an acyl group, an aryl group, or an alkoxyl group.

3. A diimmonium compound according to Claim 1 or 2, wherein R₉ and R₁₀ of the general formula (1) are aliphatic hydrocarbon groups containing a fluorine atom.

4. A diimmonium compound according to any one of Claim 1 to 3, wherein R₉ and R₁₀ of the general formula (1) are trifluoromethyl.

5. A diimmonium compound according to any one of Claim 1 to 4, wherein at least one of R₁ to R₈ of the general formula (1) is an aliphatic hydrocarbon group comprising a cyano group.

6. A diimmonium compound according to any one of Claim 1 to 4, wherein at least one of R₁ to R₈ of the general formula (1) is an aliphatic hydrocarbon group comprising a cyano group and at least one of them is an aliphatic hydrocarbon group comprising no cyano group.

7. A diimmonium compound according to any one of Claim 1 to 4, wherein at least one of R₁ to R₈ of the general formula (1) is an aliphatic hydrocarbon group comprising a halogen atom.

8. A diimmonium compound according to any one of Claim 1 to 4, wherein at least one of R₁ to R₈ of the general formula (1) is an aliphatic hydrocarbon group comprising an alkoxy group.

9. A diimmonium compound according to any one of Claim 1 to 4, wherein at least one of R₁ to R₈ of the general formula (1) is an aliphatic hydrocarbon group comprising an aryl group.

10. A diimmonium compound according to any one of Claim 1 to 4, wherein at least one of R₁ to R₈ of the general formula (1) is a C1 to C6 straight chain alkyl group.

11. A diimmonium compound according to any one of Claim 1 to 4, wherein at least one of R₁ to R₈ of the general formula (1) is a C1 to C3 straight chain alkyl group.

12. A diimmonium compound according to any one of Claim 1 to 4, wherein at least one of R₁ to R₈ of the general formula (1) is a branched alkyl group.

13. A diimmonium compound according to any one of Claim 1 to 4, wherein all of R₁ to R₈ of the general formula (1) are alkyl groups branched at terminals.

14. A diimmonium compound according to any one of Claim 1 to 4, wherein R₁ to R₈ of the general formula (1) are iso-butyl or iso-amyl.

15. A diimmonium compound according to any one of Claim 1 to 4, wherein at least one of R₁ to R₈ of the general formula (1) is an unsaturated aliphatic hydrocarbon group.

16. A composition containing the diimmonium compound according to any one of Claim 1 to 15.

17. A near-IR absorption filter comprising a layer containning the diimmonium compound according to any one of Claim 1 to 15.

18. A near-IR absorption filter for plasma displays comprising a layer containing the diimmonium compound according to any one of Claim 1 to 15.

19. An optical information recording medium comprising a recording layer containing the diimmonium compound according to any one of Claim 1 to 15.

20. An optical information recording medium comprising a recording layer containing the diimmonium compound according to any one of Claim 1 to 15 and an organic dye selected from a group consisting of cyanine type dyes, squarylium type dyes, indoaniline type dyes, and polymethine type dyes.

21. A resin composition containing the diimmonium according to any one of Claim 1 to 15.

22. A near-IR absorption compound being a salt comprising a cation obtained by oxidizing a compound having the following general formula (2) and an anion, wherein the anion is an anion defined by the following general formula (3) and necessary for neutralizing the cation; 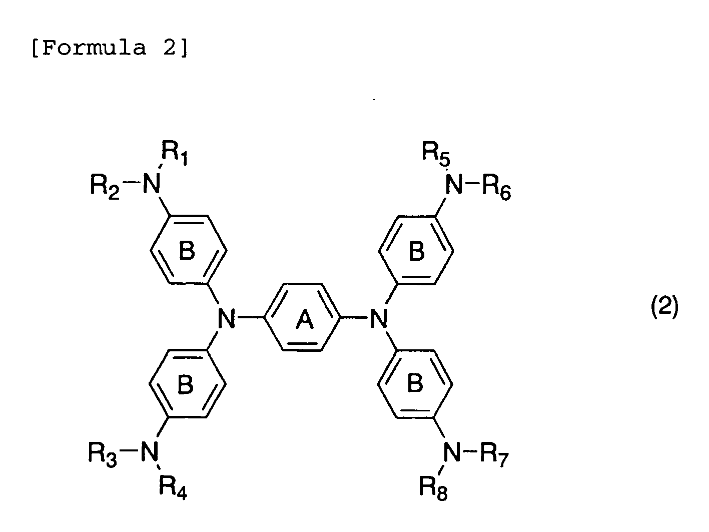 wherein R₁ to R₈ independently denote hydrogen atom or an optionally substituted aliphatic hydrocarbon group; and rings A and B may further have substituent groups; 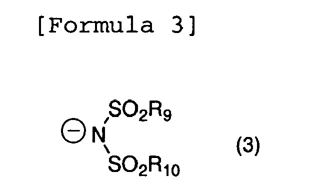 wherein R₉ and R₁₀ independently denote an aliphatic hydrocarbon group optionally containing a halogen atom.

23. A diimmonium compound having the following general formula (5) 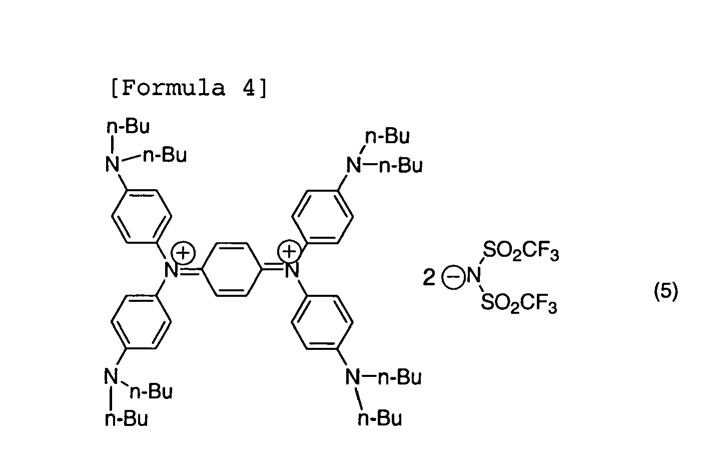 wherein n-Bu stands for n-butyl.

24. A diimmonium compound according to claim 23 having the maximum absorption wavelength (λmax) (measured in dichloromethane) of 1,102 nm.

25. A diimmonium compound having the following general formula (6) 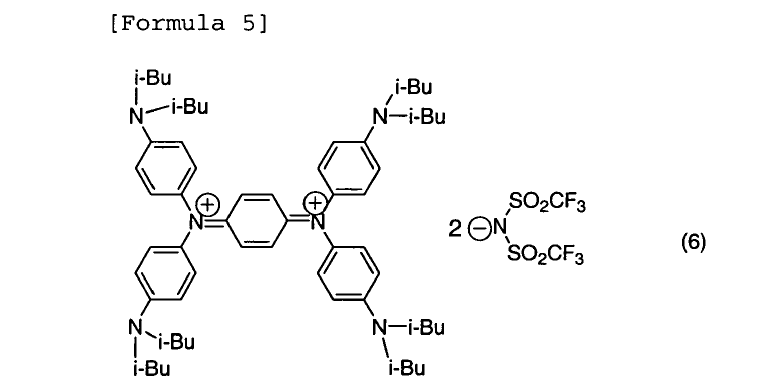 wherein i-Bu stands for iso-butyl.

26. A process for the preparation of a diimmonium compound having the following general formula (1) 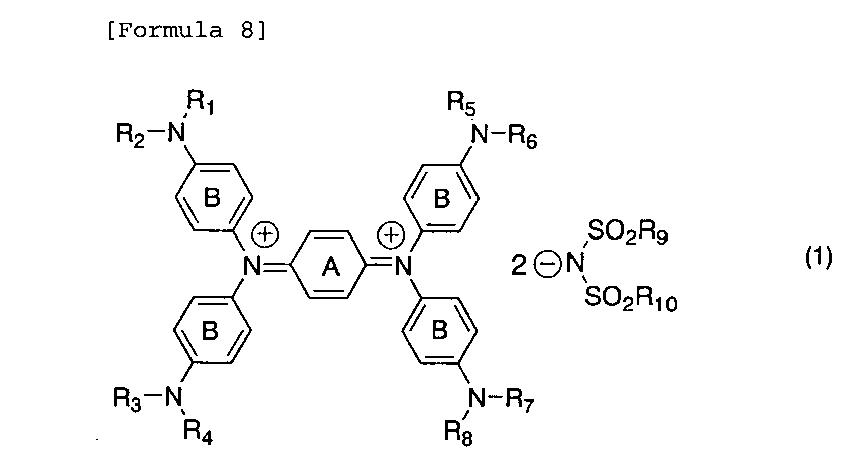 wherein R₁ to R₈ independently denote hydrogen atom or an optionally substituted aliphatic hydrocarbon group; R₉ and R₁₀ independently denote an aliphatic hydrocarbon group optionally containing a halogen atom; and rings A and B may further have substituent groups, by carrying out oxidation reaction by adding a silver salt of a mineral acid and an alkali metal salt of an anion having the following general formula (3) 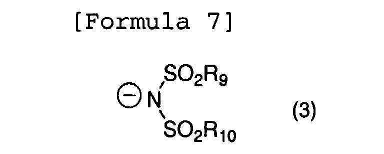 wherein R₉ and R₁₀ are the same as defined above, to a phenylenediamine compound having the following general formula (2) 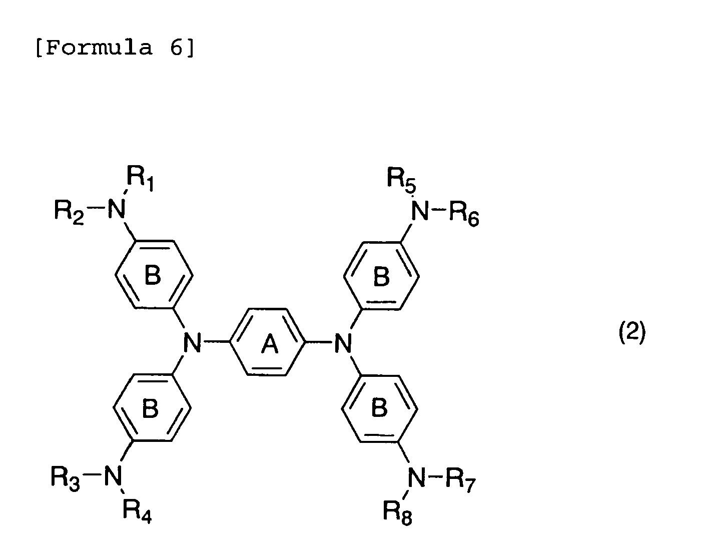 wherein R₁ to R₈ and rings A and B are the same as defined above.

27. A process for the preparation of a diimmonium compound according to Claim 26, wherein all of R₁ to R₈ are the same group selected from a group consisting of ethyl, n-butyl, iso-butyl, iso-amyl, 3-cyano-n-propyl, and 4-cyano-n-butyl and R₉ and R₁₀ are both trifluoromethyl.

28. A process for the preparation of a diimmonium compound according to Claim 26 or 27, wherein the silver salt of a mineral acid to be used is silver nitrate and the alkali metal salt of the anion to be used is a potassium salt.

29. A process for the preparation of a diimmonium compound according to any one of Claim 26 to 28, wherein the reaction is carried out in a water-soluble polar solvent.
